# EUROPEAN PATENT APPLICATION

(11) **EP 2 014 272 A1**
(43) Date of publication of application: **14.01.2009**
(21) Application number: 07110644.7
(22) Date of filing: 20.06.2007
(51) Int. Cl.: A61H 33/00, A61H 35/00

(54) **Treatment system**

(71) Applicant: Maqua Wellness Ltd., Hadley Suffolk IP7 5EA (GB)
(72) Inventor: Herrmann, Christina, 14552 Wildenbruch (DE); Berg, Dieter, 56505 Altenahr-Kreuzberg (DE)
(74) Representative: Neuefeind, Regina

(57) **Abstract**

A device for providing electromagnetic therapy and massage. The treatment device includes a magnetic field inductor for producing an electromagnetic field, and at least one magnetic micro- or nanoparticle component. The magnetic micro- or nanoparticle component includes central magnetic cores, the magnetic cores preferably being covered by a nonmagnetic material, wherein the electromagnetic field is capable of causing the magnetic micro- and/or nanoparticles to move freely and randomly within the electromagnetic field so as to massage a body or body part placed within the electromagnetic field.

## Description

A device for providing electromagnetic therapy and massage. The treatment device includes a magnetic field inductor for producing an electromagnetic field, and at least one magnetic micro- or nanoparticle component. The magnetic micro- or nanoparticle component includes central magnetic cores, the magnetic cores preferably being covered by a nonmagnetic material, wherein the electromagnetic field is capable of causing the magnetic micro- and/or nanoparticles to move freely and randomly within the electromagnetic field so as to massage a body or body part placed within the electromagnetic field.

### FIELD AND BACKGROUND OF THE INVENTION

The present invention relates to a physiotherapeutic system and, more particularly, to a system that uses a pulsating magnetic field for induced massage and for vibration to create wellness combined with pulse magnetic field therapy. Such a system finds use in the treatment of individuals with a range of clinical problems including vascular, orthopedic, rheumatologic, neurologic, and dermatologic disorders as well as in massage for preventive wellness treatments.

One major indication is peripheral vascular diesease, particularly of lower extremities. Lower extremity arterial disease (LEAD) is identified clinically by intermittent claudication an/or absence of peripheral pulses in the lower legs and feet, representing decreased arterial perfusion of the extremity. Lower extremity ulcers or even amputations are an increasing problem among individuals, e.g. suffering from diabetes.

The beneficial therapeutic effects of massage and pulsating fields individually have been well known to medicine for many years, however, previous attempts to combine them into a single therapeutic apparatus have been limited. Pulse Electromagnetic Field Therapy (PEMF) is a non-invasive treatment that works by permeating the body with a pulsed magnetic field, reaching every cell and changing cell potentials, and influencing the activity of enzymes and coenzymes by stimulating paramagnetic ions. The application of an external electromagnetic field causes the ions (charged atoms) always present in the body to travel outward toward the sides of the blood vessel walls. This increased blood flow brings more oxygen and nutrients to the cells, removing metabolic by-products from the tissues and promoting alkaline reactions (i.e., pH balance). For instance, in an ailing limb the application of the electromagnetic field speeds up recovery by improving blood flow. Therapy with an electromagnetic field has been found to be particularly effective in dermatology for treatment of such clinical problems like decubitus ulcers, skin necrosis, bums, cicatrisation defects, and the like, as well as in rheumatology, pain therapy, physiatrics, sports medicine, rehabilitation, and orthopedics, and independently in wellness applications. Since there are many documented scientific results to prove its efficiency in various conditions this electromagnetic therapy method is considered as complementary medicine. It has to be distinguished from magnet therapy (also known as biomagnetic therapy) which usually refers to a constant magnitude and vector magnetic field, created by permanent magnets. The practioners of this technology usually have a preference for one magnetic pole regarding its expected influence on the human or animal body or body parts. This therapy is considered alternative medicine.

The following discussion of PMEF is based on an analysis of the scientific basis of PMEF by Ramey (Ramey, D.W. (1998) Analysis - Magnetic and Electromagnetic Therapy, in: The Scientific Review of Alternative Medicine, Prometheus Books).

Extracellular matrix synthesis and repair are subject to regulation both by chemical agents (e.g. cytokines and growth factors) and physical agents, normally mechanical or electrical stimuli. The precise nature of such electromechanical signals is not known, however. In bone, mechanical and electrical signals may regulate the synthesis of extracellular matrix by stimulating pathways at the cell membrane (Davidovitch, Z., et al., Biochemical mediators of the effects of mechanical forces in electric currents on mineralized tissue, Calcif. Tissue Int. 36, 79-86, 1984; and Aaron, R., and Ciombor, D., Acceleration of Experimental Endochondral Ossification by Biophysical Stimulation of the Progenitor Cell Pool, J. Orthop. Res. 14(4), 582-589, 1996). In soft tissue, alternating current electrical fields induce a redistribution of integral cell membrane proteins which, hypothetically, could initiate signal transduction cascades and cause a reorganization of cytosceletal structures (Cho, M., et al., Reorganization of microfilament structure induced by ac electrical fields, FASEB J., 10, 1552-1558, 1996).

The most widely studied application of electromagnetic field therapy in human medicine is in fracture therapy. Although the mechanisms remain undetermined, several studies report that electrical fields generated by pulsating electromagnetic fields stimulate biological processes pertinent to osteogenesis (Shimizu, T., et al., Bone ingrowth into porous calcium phosphate ceramics - influence of pulsating electromagnetic fields, J. Orthop. Res. 6, 248-258, 1988; Rubin, C., McLeod, K., and Lanyoon, L., Prevention of osteoporosis by pulsed electromagnetic fields, J. Bone Joint Surg. 71, 411-416, 1989; and Cruess, R., and Bassett, C.A.L., The effects of pulsing electromagnetic fields on bone metabolism in experimental disuse osteoporosis, Clin. Orthop. 173, 345-350, 1983). This form of therapy is approved for the treatment of delayed and non-union fractures in humans in the U.S. by the United States Food and Drug Administration. Effectiveness of the treatment is supported by at least two double-blind studies (Sharrard, W., A double blind trial of pulsed electromagnetic fields for the delayed union of tibial fractures, J. Bone Joint Surg. 72, 347-355, 1990; and Mooney, V., A randomized double blind prospective study of the efficacy of pulsed electromagnetic fields for interbody lumbar fusions, Spine 15, 708-712, 1990).

Pulsating electromagnetic field therapy has also been evaluated in the treatment of soft tissue injuries with the results of some studies providing evidence that this form of therapy may be of value in promoting healing of chronic wounds, such as decubitus ulcers (Ieran, M., et al., Effect of low frequency pulsing electromagnetic fields on skin ulcers of venous origin in humans: A double-blind study, J. Orthop. Res. 8(2), 276-282, 1990), in neuronal regeneration (Kort, J., Ito, H., and Bassett, C.A.L., Effects of pulsing electromagnetic fields in peripheral nerve regeneration, J. Bone Jt. Sug. Orthop. Trans. 4, 238, 1980; and Sisken, B.F., et al., Pulse electromagnetic fields stimulate nerve regeneration in vitro and in vivo, Restorative Neurology and Neuroscience 1, 303-309, 1990), and in many other soft tissue injuries (Polk, C., Electric and magnetic fields for bone and soft tissue repair, in: Handbook of Biological Effects of Electromagnetic Fields, 2nd ed., Polk, C., and Postow, E. (eds.), CRC Press, Boca Raton, FL, 231-246, 1996; and Bassett, C.A.L., Beneficial effects of electromagnetic fields, J. Cell Biochem. 51, 387-393, 1993).

The direct mechanical effect of rhythmically applied pressure by massage also increases the blood flow rate. It stimulates the nerve receptors and causes the blood vessels to dilate, allowing increasing fluid exchange (e.g. blood, oxygen and lymph, and the removal of waste products such as lactic acid and carbon dioxide). Massage technology is well established with universal acceptance of its efficacy. Massage has proven beneficial effects in many fields, including orthopedics, traumatology, rheumatology, treatment of complicated and simple fractures, treatment of wounds, bums and degenerative diseases, coronary and circulation diseases and disorders of the neurological system. Massage therapy may be applied in any of several ways ranging from manual treatment by a massage therapist to the use of a mechanical device to assist in the application of pressure and motion.

It would be desirable to have a therapeutic system that combines the beneficial effects of magnetic field therapy massage and massage for the prevention, alleviation and/or treatment of circulatory disorders such as peripheral arterial disease and its complications as well as for other medical preventions, alleviations and/or treatments for use with humans or non-human animals.

A number of devices have been previously described for generating an electromagnetic field for providing electromagnetic therapy, and are disclosed, e.g., in U.S. Patent No. 4,066,065 issued to Kraus, U.S. Patent no. 4,765,310 issued to Deagle et al.*,* U.S. Patent No.4,838,850 issued to Rosengart, U.S. Patent No. 5,088,661 issued to Davidson et. al., WO 03/006,102 issued to Salkiner et al.*.* Similarly, a number of patents disclose mechanical devices for assisting in providing massage therapy, icluding, e.g., U.S. Patent no. 3,585,990 issued to Blachly et al*.,* U.S. Patent No. 3,636,945 issued to Sato, U.S. Patent no. 5,123,406 issued to Masuda, and U.S. Patent No. 5,462,515 issued to Tseng.

U.S. Patent No. 6,102,875 issued to Jones teaches a handheld apparatus for simultaneously applying massage and biomagnetic therapy. This device uses a permanent magnet of fixed rather than alternating polarity. Massage balls are attached to the head of the device and are rotated by the action of an electric motor. An electromagnetic or electromechanical vibrator can impart vibrational motion to the balls. Such a device suffers from a number of limitations. It is handheld and imparts a local effect only on the limited portion of the body which is placed in contact, it imparts only kneading and vibrational massage and it utilizes fixed biomagnetic therapy rather than a pulsed electromagnetic field.

Another combination device is that taught in U.S. Patent No. 5,084,003 issued to Susic. That device includes a coil that generates an electromagnetic field and small permanent magnetic plates arranged inside a cover. The plates undulate or vibrate to provide a massage effect. Such a device suffers from several limitations.
The magnetic plates are fixed in a particular arrangement and thus only limited movement of the plates is possible, imparting only a vibrational, flat type of massage. The device includes a bed and is not readily mobile but rather stationary.

Thus there is a widely recognized need for a therapeutic system that combines massage and pulse magnetic field therapy devoid of the above limitations.

Patent No. WO 03/006,102 issued to Salkiner et al*.* teaches an improved apparatus for simultaneously applying massage and biomagnetic therapy. Larger size magnetic balls are applied in a pulsating electric field and an improved safety system is already described. This system, however, suffers still from various disadvantages. The magnetic balls are relatively large in size and thus the intensity of massage effects has to be improved. Also the equipment is specialized und thus restricted to medical applications in a special apparatus.

Thus there is a widely recognized need for a therapeutic and wellness system that combines massage and pulse magnetic field therapy and can also be used for wellness purposes. This wellness system has been specifically designed to allow easy dissemination and therefore can be applied using common acrylic bathtubs to benefit from the effects of magnetic and/or magnetically susceptible micro- and/or nanoparticles delivering more intensive massage and therapy results.

### SUMMARY OF THE INVENTION

According to the present invention a device is described that provides electromagnetic therapy and massage (also for preventive and wellness purposes) including (a) an electromagnetic field inductor for producing an electromagnetic field, and (b) magnetic micro- and/or nanoparticles which may have a central magnet core covered in a substantionally nonmagnetic material, wherein the electromagnetic field is capable of causing the magnetic micro- and/or nanoparticles to move freely and randomly within the electromagnetic field so as to massage a full body or body part placed within the magnetic field of the system.

According to further features in preferred embodiments of the invention as described below, the electromagnetic field has a maximal induction of 200 Gauss.

According to further features in preferred embodiments of the invention as described below, the electromagnetic field is pulsed.

According to further features in preferred embodiments of the invention as described below, the electromagnetic field can also be intermittent.

According to further features in preferred embodiments of the invention as described below, the electromagnetic field is alternating in polarity.

According to further features in preferred embodiments of the invention as described below, the electromagnetic field alternates in polarity with a frequency of 20 - 20,000 Hertz.

According to still further features in preferred embodiments of the invention as described below, the electromagnetic field alternates in polarity with sinusoidal oscillations.

According to still further features in preferred embodiments of the invention as described below, the body or body part is of an animal.

According to still further features in preferred embodiments of the invention as described below, the body or body part is of a human.

According to still further features in preferred embodiments of the invention as described below, the body part is a bone, e.g. a limb.

According to still further features in preferred embodiments of the invention as described below, the magnetic material is micro- and/or nanoparticles.

According to still further features in preferred embodiments of the invention as described below, the substantially nonmagnetic material is soft.

According to still further features in preferred embodiments of the invention as described below, the substantially nonmagnetic material is hard.

According to still further features in preferred embodiments of the invention as described below, the shape of the micro- and/or nanoparticles is selected from the group consisting of spheres, cubes, cylinders, cones, pyramids, rectangular prisms, irregular polyhedral solids, and a mixture thereof.

According to still further features in preferred embodiments of the invention as described below, particles are nanoparticles and/or microparticles. These particles are from 1 nanometer up to 1,000 micrometer in size.

According to still further features in preferred embodiments of the invention as described below, the substantionally nonmagnetic material is selected from the group consisting of plastic, rubber, silicone, silicone epoxy, foam rubber, fabric, and polyelectrolytes.

According to still further features in preferred embodiments of the invention as described below, the device further comprises a housing, the housing comprising at least one wall and a base surface, the at least one wall and the base surface enclosing a bath, wherein the electromagnetic field inductor surrounds the bath, and the magnetic material moves freely and randomly within the bath.

According to still further features in preferred embodiments of the invention as described below, the device further may include a cover above the bath, the cover preferably having anaperture therein for insertion of the body or the body part of an animal or human, the aperture more preferably being located in the center of the device.

According to still further features in preferred embodiments of the invention as described below, the electromagnetic field inductor is circular and/ or variable in design.

According to still further features in preferred embodiments of the invention as described below, the device further includes a control element for automated operation of the device.

According to still further features in preferred embodiments of the invention as described below, the control element is programmable, such that at least one parameter of the operation of the device may be changed by the operator of the device.

According to still further features in preferred embodiments of the invention as described below, the at least one paramenter is selected from the group consisting of maximal intensity of the electromagnetic field, frequency and/or frequency variation of the electromagnetic field, pulse cycle time, and total treatment duration.

According to still further features in preferred embodiments of the invention as described below, the at least one parameter may be changed using at least one remote program input device connected to the control element by at least one communication channel.

According to still further features in preferred embodiments of the invention as described below, the at least one communication channel is selected from the group consisting of a telephone connection, a cellular telephone connection, an infrared connection, a satellite connection, cables connection, an Internet connection, a local area network connection, a radio frequency connection, and a varying and variable frequency connection of any other origin.

According to still further features in preferred embodiments of the invention as described below, the control element is adapted to perform a stop, the stop more preferably being an emergency stop. The device may comprise a remote input device for causing the control element to perform the stop. The control element may also be adapted so as to prevent operation of the device, if at least one safety parameter is exceeded.

According to still further features in preferred embodiments of the invention as described below, at least one safety parameter is selected from the group consisting of inductor temperature and current intensity.

According to still further features in preferred embodiments of the invention as described below, the system is used for treatment of vascular disease.

According to still further features in preferred embodiments of the invention as described below, the system is used for the treatment of inflammatory conditions.

According to still further features in preferred embodiments of the invention as described below, the system is used for the treatment of pain.

According to still further features in preferred embodiments of the invention as described below, the system is used for wellness massages.

According to still further features in preferred embodiments of the invention as described below, the system is used for enhancement of wellness feeling.

The present invention successfully addresses the shortcomings of the presently known configurations by providing a system that uses a pulsed electromagnetic field for induced massage with random percussion combined with pulse magnetic field therapy in medicine, massage and/or wellness systems.

According to the present invention, magnetic and/or magnetically susceptible micro-and/or nanoparticles are used in an electromagnetic field for the provision of a treatment system for massaging a body or a body part of an animal or a human and/or for alleviating, preventing and/or combating vascular diseases, inflammatory conditions, pain and/or dermal infections.

### BRIEF DESCRIPTIONS OF THE DRAWINGS

This invention is herein described, by way of example only, with reference to the accompanying drawings. With specific reference now to the drawings, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention that it is necessary for a fundamental understanding of the invention, the description taken with the drawings making apparent to those skilled in the art how several forms of the invention may be embodied in practice.

In the drawings:
Fig. 1 is a schematic drawing illustrating the principles and activity of the present invention;
Fig. 2 is a simplified block diagram showing the general components of a preferred embodiment of the system according to the present invention;
Fig. 3 is a perspective view of a preferred embodiment of the system of the present invention;
Fig. 4 is a schematic longitudinal cross-section illustrating a preferred embodiment of the system of the present invention;
Fig. 5 is a schematic diagram of a preferred embodiment of a micro- and/or nanoparticle according to the present invention.

### DESCRIPTION OF THE PREFERRED EMBODIMENTS

The present invention describes a device that uses a pulsed electromagnetic field for induced random percussion massage combined with pulsed magnetic field therapy that can be used for physiotherapeutic treatment as well as for wellness enhancement. Specifically, the present invention can be used to increase circulatory flow in the treatment of vascular diseases, e.g. diabetes mellitus induced peripheral vascular disease.

The principles and operation of a magneto-massage and wellness system according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings, The invention is capable of other embodiments or of being practiced or carried out in various ways. Also it is understood that the phraseology and terminology used herein is for the purpose of description and shall not be regarded as limiting.

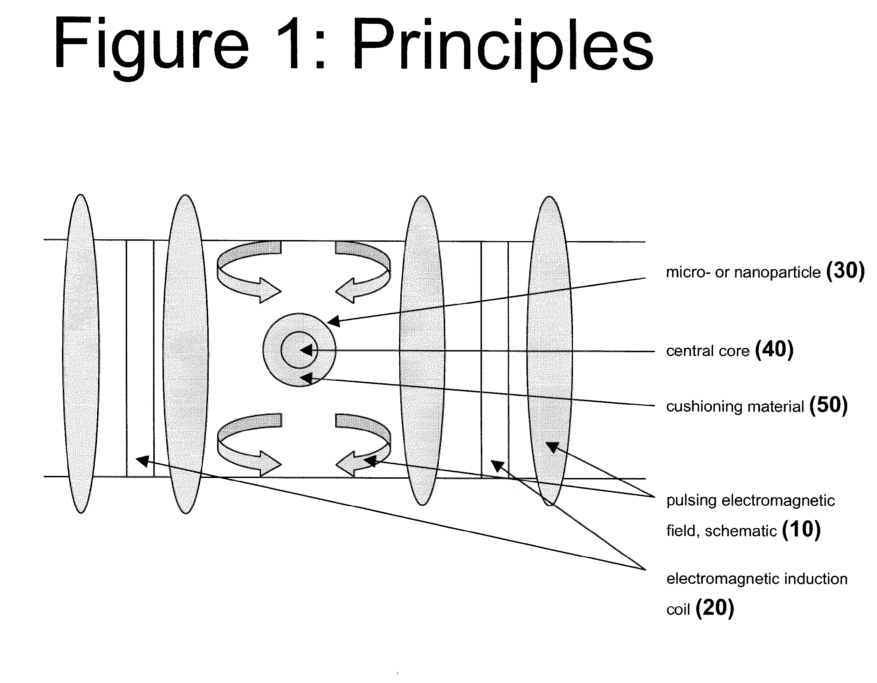

Referring now to the drawings, Fig. 1 illustrates the principles underlying the device of the present invention. A relatively strong pulsed electromagnetic field **(10)** is created by an electromagnetic induction coil **(20).** Within field **10** a plurality of massage micro- and/or nanoparticles is placed, each of which has a central core **(40)** composed of a magnet, the central core preferably being covered with a cushioning material **(50)**. The electromagnetic field **10** is pulsed and preferably, though not necessarily, alternating. Electromagnetic field **10** thus has a rapidly changing polarity producing shifting vectors of changing magnitude and in some cases direction to apply attraction and repulsion to magnet **(40)**. The pulsing magnetic field thus causes the micro- and/or nanoparticles **30** within the field (whose magnetic polarity is randomly oriented) to move freely and randomly and to change movement direction and speed very frequently. Electromagnetic field **10** can also be intermittent in certain preferred embodiments, and therefore sometimes is present ("on") and sometimes is absent ("off") at changeable controllable duty cycles. During the "off" periods, the effects of other forces such as gravity acting on the micro- and/or nanoparticles **30** becomes greater, further contributing to the randomness of position and orientation of the central magnet core **40** and the micro-and/or nanoparticles **30.** Massage micro- and/or nanoparticles **30** impact on and bounce off coil **20** and any other object placed within field **10**. A human or animal body or body part may be placed within field **10**. The body or body part positioned within field **10** will thus receive the therapeutic effects of pulsed electromagnetic field **10** as well as will have applied a percussion massage through the effect of the physical contact of micro- and/or nanoparticles **30** impacting upon the body or body part.

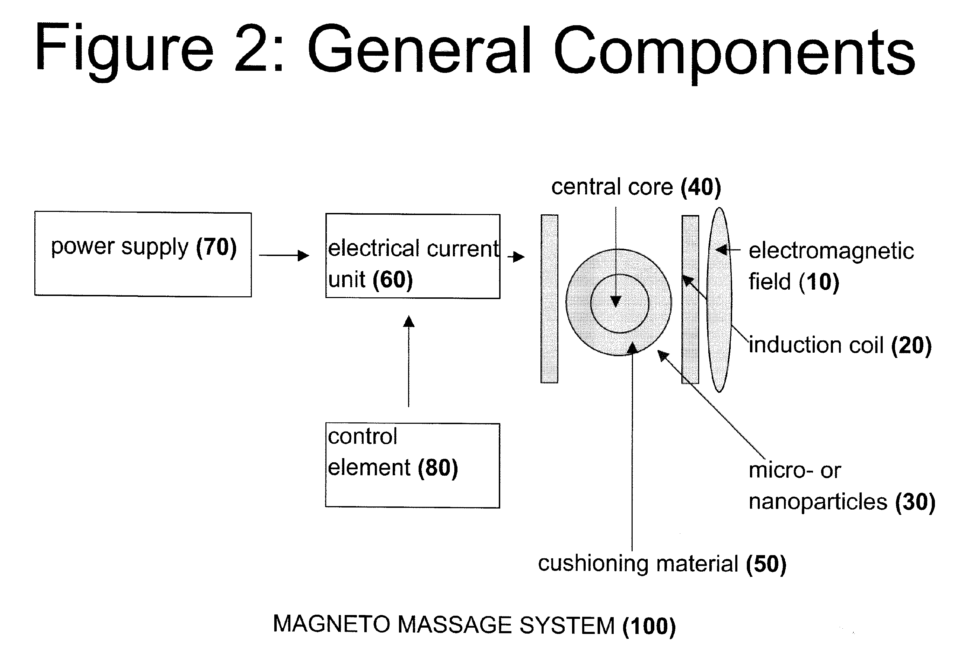

Fig. 2 is a simplified block diagram showing the general components of a preferred embodiment of the magneto-massage and wellness system **(100)** according to the present invention. Electromagnetic field **10** is produced by electromagnetic field induction coil **20** that is connected to pulsed electrical current circuit **(60)**. Circuit **60** produces pulses of alternating phase controlled current such that electromagnetic field **10** produced by coil **20** is pulsed varying in frequency, magnitude and intensity and preferably, but not necessarily, alternating in polarity. Alternate embodiments in which electromagnetic field **10** produced by coil **20** is pulsed but not alternating in polarity are envisioned as being within the scope of the present invention. The design and operative assembly of circuit **60** and coil **20** are done in accordance with principles commonly known to those of ordinary skills in the art to which this invention pertains. Circuit **60** is further connected to power supply (**70**) and control element (**80**) that controls the automated and programmable operation of the entire system **100**. In a preferred embodiment of the present invention, described herein, circuit **60** does not only utilize a current generator or oscillator, but also may draw the alternating current directly from a 50-60 Hertz main power line. Electromagnetic field **10** acts on the massage micro-and/or nanoparticles **30** placed within field **10,** each of which has a central core **40** composed of a magnet, the central core preferably being covered with a substantially nonmagnetic cushioning material (**50**). In certain alternative embodiments, the central core magnet **40** can be a permanent magnet, while in others, central core magnet **40** is composed of a magnetically susceptible material such as e.g. iron that becomes magnetic when placed within an electromagnetic field. Thus central core magnet **40** is at least composed of a magnetic susceptible material such that this material becomes magnetized in an electromagnetic field acting thereon.

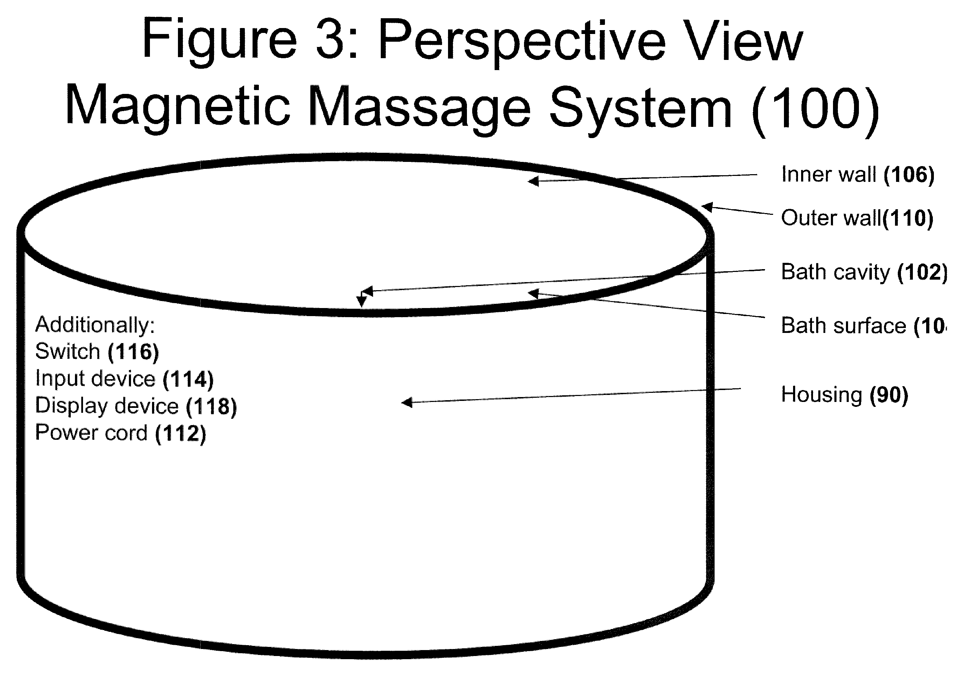

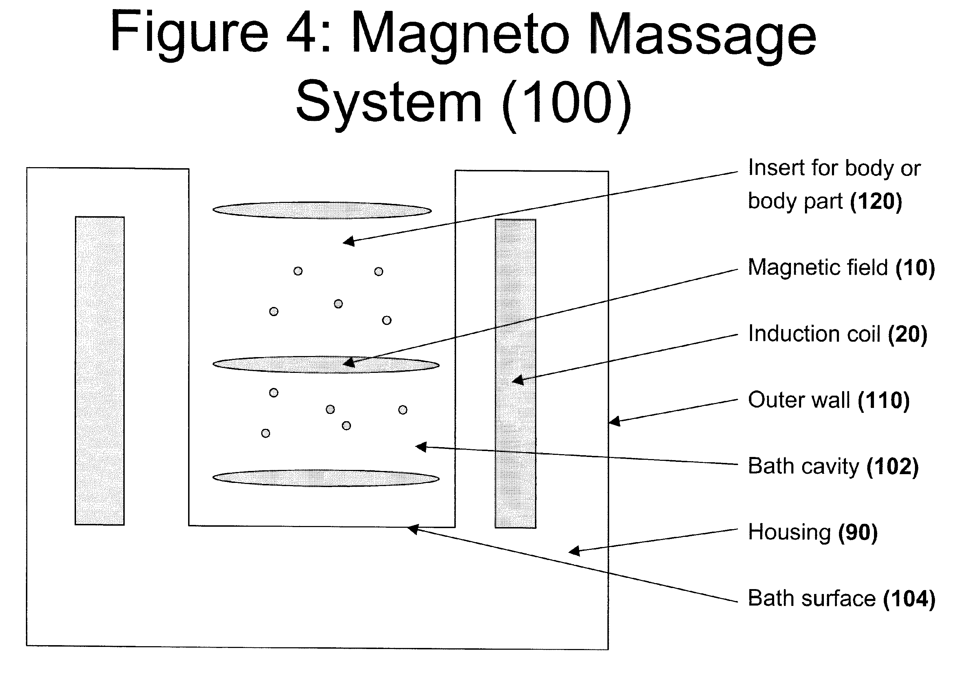

Fig. 3 is a perspective view of a preferred embodiment of the system of the present invention, and Fig.4 is a schematic longitudinal cross-section illustrating a preferred embodiment of the system of the present invention. The components of system **100** are contained within a housing **90** of a durable material such as e.g. PVC plastic or acrylate polymers, but not restricted to these. Housing **90** contains a base surface **104** and inner walls **106** that enclose a bath cavity **102** defining a bath. The upper surface of bath cavity **102** is open. While for illustrative purposes housing **90** is shown in Fig. 3 and 4 as an oblong rectangular prism, housing **90** and bath **102** may be of varying shapes and sizes. Induction coil **20** is located within the housing **90**, between inner walls **106** and outer walls **110**, surrounding bath cavity **102**, such that electrical field **10** is produced within bath **102**. Power for the system can be provided by connection of system to main supply or power via power cord **112**. An operator, who may be the person being treated or another individual such as a physician therapist, physician, nurse, or a wellness advisor, controls system **100** through at least one input device such as button **114** and switch **116**. Operation of the system can be monitored using at least one display device, such as light **118**.

Micro- and/or nanoparticles **30** are placed freely in no particular specified arrangement within bath **102**. Micro- and/or nanoparticles **30** are agitated into random motion by the effects of the pulsed electromagnetic field **10**. A body or body part **120** to be treated is placed into bath **102**, and thus into field **10**. The body or the part thereof may be that of a human or an animal. When the system is activated, in addition to the electromagnetic field acting upon the tissues of body or body part **120**, micro- and/or nanoparticles **30**, moving freely and randomly, will impact surfaces **104** and **106** of the system. The physical contact of micro-and/or nanoparticles **30**, directly or indirectly, with body or body part **120** will impart percussion massage on body or body part **120**. The frequency, force and speed of the impact and the quality of the massage can be changed by altering various parameters, including those of field **10** and of micro- and/or nanoparticles **30**.

Altering the magnitude and strength of the field and the frequency of oscillation for example can influence the speed and frequency of the contact. The quality of the massage can be further altered by a change in the number and sizes of particles **30**. In addition other characteristics of micro- and/or nanoparticles **30** can be varied in order to alter the quality of the massage.

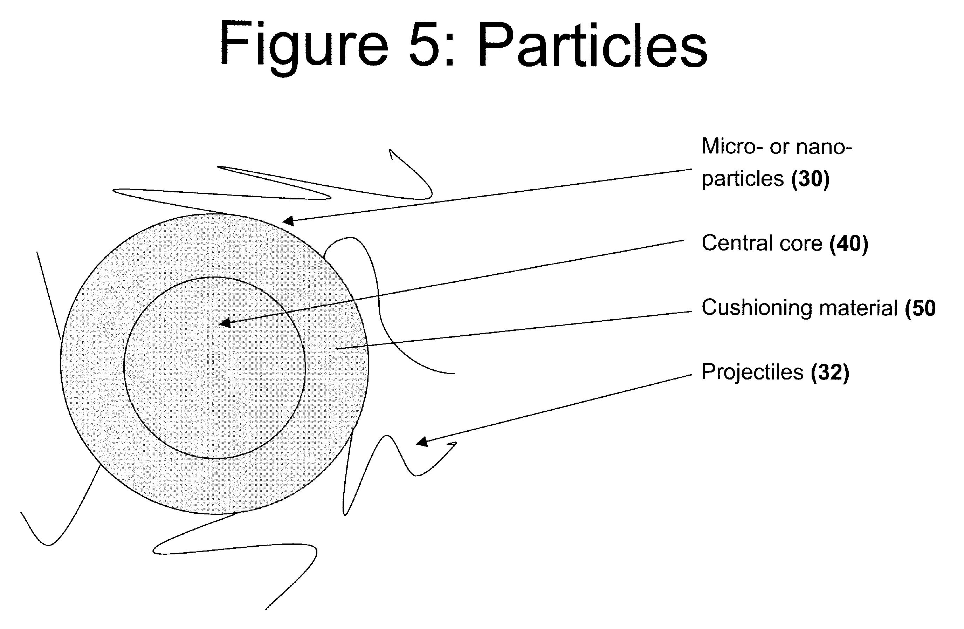

For the purpose of the specification and accompanying claims, use of the term "micro-and/or nanoparticles" is meant to encompass projectile objects. It will be appreciated that micro- and/or nanoparticles **30** could be either of spherical shapes or of non-spherical shapes, including, but not limited to, cubes, cylinders, cones, pyramides, rectangular prisms, plates, or irregular polyhedral solids. Further encompassed by the term "particles" are objects whose surfaces have projections (**32**) radiating and extending from the surface, including spikes, bumps, and knobs (see Fig. 5). In those embodiments with a plurality of micro- and/or nanoparticles **30**, regardless of the shape is that they consist of a magnetic core **40,** made for example of a ferromagnetic material of any varying shape, the magnetic core preferably being covered by some other material **(50)**. In certain alternative embodiments, the central core magnet **40** is composed of a magnetically susceptible material (such as iron) that becomes magnetic when placed within an electromagnetic field.

Covering material **50** used is variable and in different configurations is soft or hard, and rigid or flexible. Covering material **50** may be produced from any of a number of generally synthetic, generally non-conductive materials including polyelectrolytes, plastics (hard or soft), rubber, silicone epoxies, foam rubber, or fabric as non-limiting examples. The particles according to the invention are selected from the groups consisting of nanoparticles and microparticles, the particles **30** being from 1 nanometer up to 1,000 micrometer in size. Preferably, the sizes of particles **30** range from 200 nanometers to 600 micrometers, and most preferably from 800 nanometers to 400 micrometers. Particles with a size from 1 nanometer up to 1,000 nanometer are defined to be nanoparticles, and particles with a size from 1 micrometer up to 1,000 micrometer are defined to be microparticles. The number of micro-and/or nanoparticles **30** can be varied broadly and depends on the desired intensity of the massage.

All parts of system **100** including the particles, sleeves, or housing surfaces, that come into contact with the body or body part **120** to be treated are made from biocompatible materials that are well known in medical and wellness applications. All circuitry is designed to medical and/or wellness standards.

One should however take note that the massage application in which the device of the present invention is used for management and prevention from applications of diseases and not the disease processes themselves or for the wellness massages described, is by no means the sole potential use of the device of the present invention. Specifically envisioned as being within the scope of the medical applications of this invention is use of the device described herein above for other such applications, including but not limited to: diabetes mellitus secondary diseases, non-union of fractures, delayed bone healing and failed arthroses, osteoporosis, avascular necrosis of the hip, degenerative spine diseases, non-infectious inflammatory conditions including osteoarthritis and rheumatoid arthritis, soft tissue injuries, various skin conditions, and pain including chronic pain conditions.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

Citation or identification of any reference in this application shall not be construed as an admission that such reference is available as prior art to the present invention.

## Claims

1. A device for providing electromagnetic therapy, electromagnetic massage and/or wellness massage comprising,
(a) an electromagnetic field inductor for producing an electromagnetic field, and
(b) magnetic and/or magnetically susceptible micro- and/or nanoparticles, wherein the electromagnetic field is capable of causing the magnetic and/or magnetically susceptible micro- and/or nanoparticles to move freely and randomly within the electromagnetic field..

2. The device of claim 1, wherein the electromagnetic field is pulsed.

3. The device of claims 1 or 2, wherein the electromagnetic field is intermittent.

4. The device of any one of claims 1 to 3, wherein the shape of the micro- and/or nanoparticles is selected from the group consisting of spheres, cubes, cylinders, cones, pyramids, rectangular prisms, irregular polyhedral solids, and a mixture thereof.

5. The device of any one of the preceding claims, wherein the particles are of from 1 nanometer up to 1,000 micrometer in size.

6. The device of any one of the preceding claims, wherein the magnetic and/or magnetically susceptible micro- and/or nanoparticles have a central magnetic and/or magnetisable core covered in a substantially nonmagnetic material.

7. The device of claim 6, wherein the substantially nonmagnetic material is selected from the group consisting of polyelectrolytes, plastic, rubber, silicone epoxy, foam rubber and fabric.

8. The device of any one of the preceding claims, further comprising a housing, the housing comprising at least one wall and a base surface enclosing a bath, wherein the electromagnetic field inductor surrounds said bath, and the micro- and/or nanoparticles move freely and randomly within said bath.

9. The device of any one of the preceding claims, wherein the electromagnetic field inductor has a central aperture adapted to enable insertion of a body or body part of an animal or a human.

10. Use of magnetic and/or magnetically susceptible micro- and/or nanoparticles in an electromagnetic field for the provision of a treatment system for massaging a body or body part of an animal or a human and/or for alleviating, preventing and/or combating vascular diseases, inflammatory conditions, pain and/or dermal infections.
